Europäisches Patentamt

European Patent Office

Office européen des brevets

(11) Publication number: **0 096 694**
**B1**

(12) **EUROPEAN PATENT SPECIFICATION**

(45) Date of publication of patent specification: **01.03.89**

(21) Application number: **83900165.8**

(22) Date of filing: **22.12.82**

(86) International application number:
**PCT/AU82/00217**

(87) International publication number:
**WO 83/02247 07.07.83 Gazette 83/16**

(60) Divisional application 87202206 filed on 12.11.87.

(51) Int. Cl.⁴: **A 61 B 17/10, B 25 C 5/11, B 25 C 5/10**

(54) **SURGICAL STAPLING INSTRUMENT.**

(30) Priority: **22.12.81 AU 2053/81**
**28.06.82 AU 4621/82**

(43) Date of publication of application:
**28.12.83 Bulletin 83/52**

(45) Publication of the grant of the patent:
**01.03.89 Bulletin 89/09**

(84) Designated Contracting States:
**AT CH DE FR GB LI SE**

(56) References cited:
**WO-A-81/03303**
**WO-A-82/00968**
**AU-D-2 885 077**
**AU-D-7 864 581**
**GB-A- 487 085**
**GB-A-2 056 898**
**GB-A-2 093 395**
**SU-A- 266 138**
**US-A-3 269 630**
**US-A-3 949 923**

(73) Proprietor: **HOSPITAL PRODUCTS LIMITED**
**10 Clarke Street**
**Crows Nest. N.S.W. 2065 (AU)**

(72) Inventor: **FREUND, Hans Ludwig**
**208 Woniora Road**
**South Hurstville NSW 2221 (AU)**
Inventor: **KERR, Gavin**
**14 Kathleen Avenue**
**Castle Hill NSW 2154 (AU)**

(74) Representative: **Needle, Jacqueline et al**
**PAGE, WHITE & FARRER 5 Plough Place New Fetter Lane**
**London EC4A 1HY (GB)**

Courier Press, Leamington Spa, England.

**Description**

This invention relates to surgical stapling instruments of the type in which staples are ejected from a staple cartridge to be formed against an anvil surface mounted in juxtaposition with the cartridge, the staple cartridge being movable relative to the anvil between retracted and staple firing positions.

Prior art instruments of this type are well known, and are exemplified by the instrument described in U.S. Patent 3,490,675. The prior art instruments comprise, in addition to jaws or other means of supporting the anvil surface and a cartridge holder, an elongated housing containing the drive mechanism, an operating handle and means for the movement of the cartridge holder.

In US-A-3 269 630, there is disclosed a surgical stapling instrument of the kind in which staples are ejected from a staple cartridge to be formed against an anvil surface mounted in juxtaposition with said cartridge, the cartridge being movable between a staple firing position at a predetermined distance from the anvil surface and a retracted position at a greater distance from the anvil surface. The instrument comprises a cartridge holder movable to control the position of the cartridge and a staple driving member movable towards the cartridge to cause ejection of staples therefrom and an operating lever movable to cause staple ejecting motion of the staple driving member. The operating lever is operable in a first movement thereof to cause movement of the cartridge holder towards the anvil to place the cartridge in the staple firing position and is operable in a second movement thereof to cause staple ejecting motion of the driving member. This apparatus further comprises pusher means extending rearwardly from the cartridge holder and longitudinally moveable to control the position of the cartridge holder, pusher drive means actuated by the operating lever to place the cartridge holder in the staple firing position, staple driving member extension means extending rearwardly from the staple driver, and a staple firing member associated with the operating lever, the staple firing member being engagable with the extension means after actuation of the pusher drive means.

A principal object of the present invention is the provision of an instrument of this type in which adequate stapling force may reliably be applied in single-handed operation, and the provision of an actuating mechanism for such an instrument which is more convenient in operation than those of the prior art.

A further object of the invention, as applied to instruments of the type in which the anvil surface and the staple cartridge are mounted between opposed jaws, is to enable interchange of the jaws, so that jaws and cartridge/anvil assemblies of differing standard sizes (e.g. 90 mm, 55 mm, 30 mm) may be employed with the same actuating unit, thereby effecting a substantial cost saving in the hands of consumers. Other objects of the present invention, and advantages of the instrument described, will become apparent from the ensuing description.

In one broad form, the invention comprises a surgical stapling instrument of the kind in which staples are ejected from a staple cartridge to be formed against an anvil surface mounted in juxtaposition with said cartridge, said cartridge being movable between a staple firing position as a predetermined distance from said anvil surface and a retracted position at a greater distance from said anvil surface, said instrument comprising a cartridge holder movable to control the position of said cartridge, a staple driving member movable towards said cartridge to cause ejection of staples therefrom, and an operating lever movable to cause staple ejecting motion of said staple driving member, wherein said operating lever is operable in a first movement thereof to cause movement of said cartridge holder towards said anvil to place said cartridge in said staple firing position and is operable in a second movement thereof to cause said staple ejecting motion of said drive member, and pusher means extending rearwardly from said cartridge holder and longitudinally moveable to control the position of the cartridge holder, pusher drive means actuated by said operating lever to move said pusher means in a forward direction to place said cartridge holder in said staple firing position, staple driving member extension means extending rearwardly from said staple driver, and a staple firing member associated with said operating lever, said staple firing member being engagable with said extension means after actuation of said pusher drive means; the instrument being characterised in that said pusher drive means comprises a toggle having a rear link and a forward link, the forward pivot of said forward link being connected to said pusher means, the forward and rear links being interconnected by a toggle pivot, said operating lever being provided with means engaging said toggle in the region of said toggle pivot upon movement of said lever in an operating direction to expand said toggle thereby moving said pusher means forwardly.

In the accompanying drawings which are included by way of example only;

Fig. 1 shows a stapling instrument according to the present invention in side elevation;

Fig. 2 is a partly sectioned side elevation of the instrument with one housing half removed to show the operating mechanism;

Fig. 3 is a sectional plan taken on the line 3-3 of Fig. 2 with the mechanism of the instrument in its fired condition;

Fig. 4 is a side elevation of an instrument housing half showing portion of the instrument operating mechanism;

Fig. 5. is a section taken on the lines 5-5 of Fig. 4;

Fig. 6. is a cross-section taken on the line 6-6 of Fig. 4;

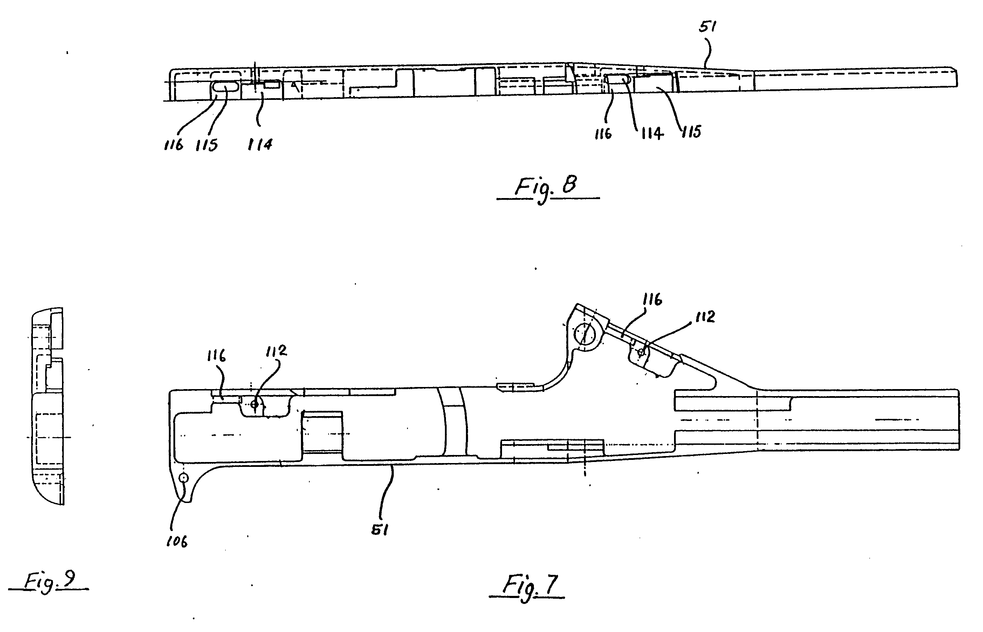

Fig. 7 is a side elevation of the other housing half of the instrument;

Fig. 8 is a plan view of the housing half illustrated in Fig 7;

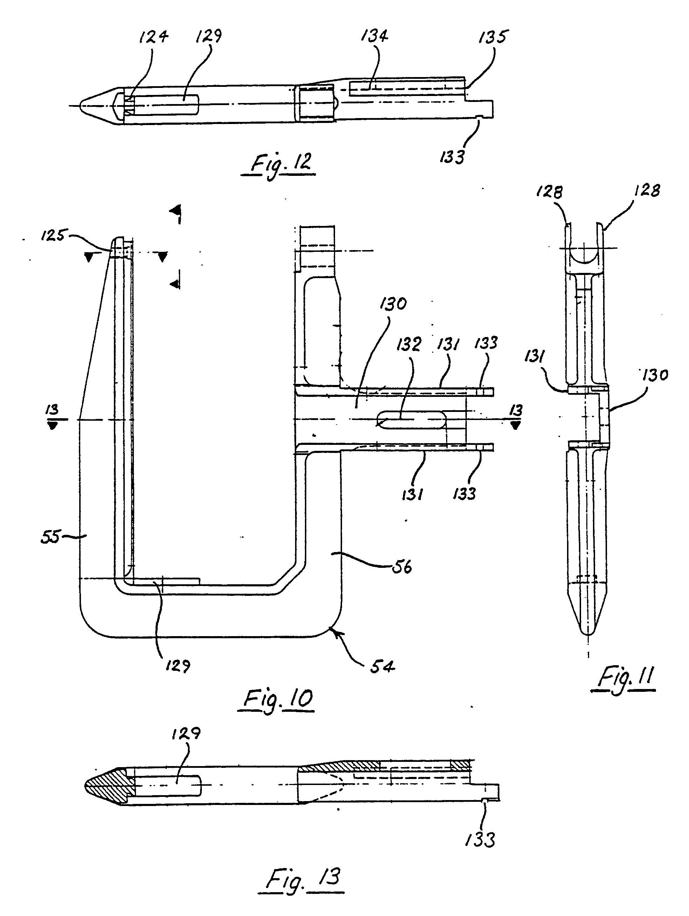

Fig. 9 is an end elevation of the housing half shown in Fig. 7;

Fig. 10 is a side elevation of a jaw suitable for use with the instrument of Figs. 1 to 9;

Fig. 11 is an end elevation of the jaw illustrated in Fig. 10;

Fig. 12 is a plan view of the jaw illustrated in Fig. 10;

Fig. 13 is a section taken on the line 13-13 of Fig. 10;

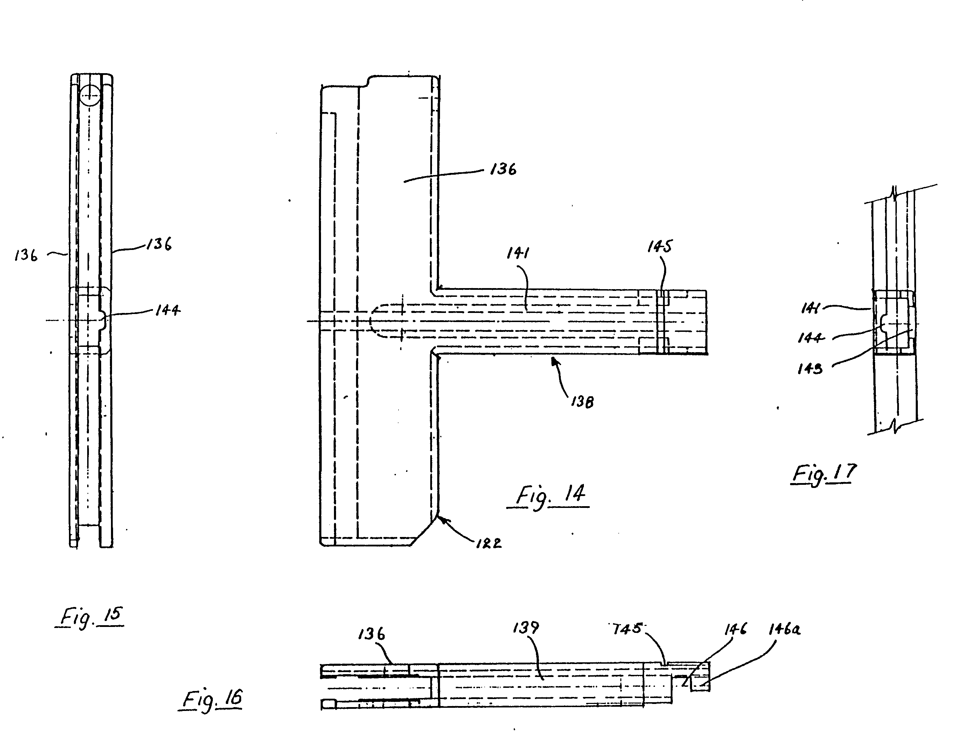

Fig. 14 is a side elevation of a cartridge holder suitable for use in the illustrated instrument;

Fig. 15 is an end elevation of the cartridge holder of Fig. 14;

Fig. 16 is a bottom plan view of the cartridge holder;

Fig. 17 is a fragmentary end elevation of the cartridge holder illustrated in Fig. 14;

Fig. 18 is a side elevation of a T-bar;

Fig. 19 is a section taken on the line 19-19 of Fig. 18;

Fig. 20 is a side elevation of a locking slide;

Fig. 21 is a plan view of the locking slide of Fig 20;

Fig. 22 is an end elevation of the locking slide;

Fig. 23 is a side elevation of the head assembly of the illustrated instrument;

Fig. 24 is an end elevation of the head assembly;

Fig. 25 is a section taken on the line 25-25 of Fig. 23;

Fig. 26 is a section taken on the line 26-26 of Fig. 23;

Fig. 27 is a side elevation of a T-bar extension;

Fig. 28 is a plan view of the T-bar extension;

Fig. 29 is a section taken on the line 29-29 of Fig. 28;

Fig. 30 is a side elevation of a pusher;

Fig. 31 is a plan view of the pusher;

Fig. 32 is an end elevation of the pusher;

Fig. 33 is a side elevation of a latch;

Fig. 34 is an end elevation of the latch;

Fig. 35 is a schematic elevation of the instrument mechanism in a first actuated position; and

Fig. 36 is a schematic elevation of the mechanism in a second actuated position.

BEST MODE FOR CARRYING THE INVENTION

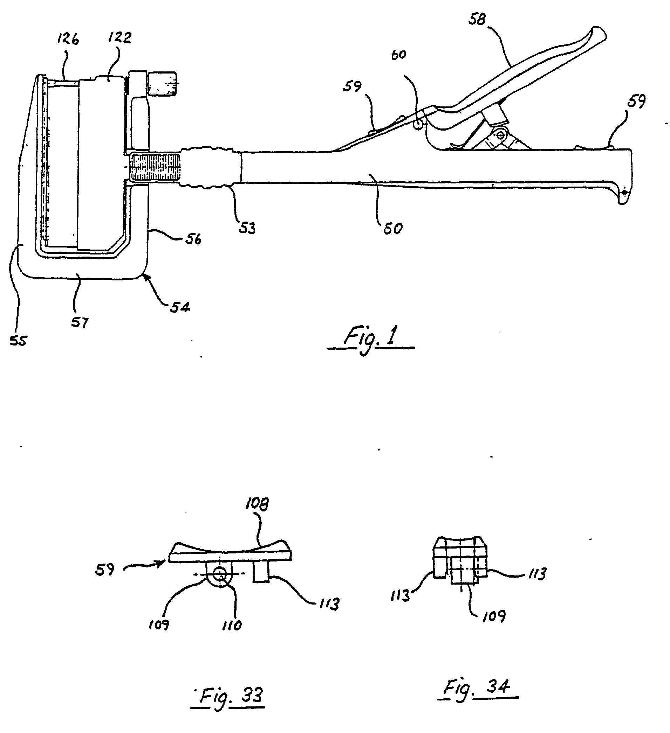

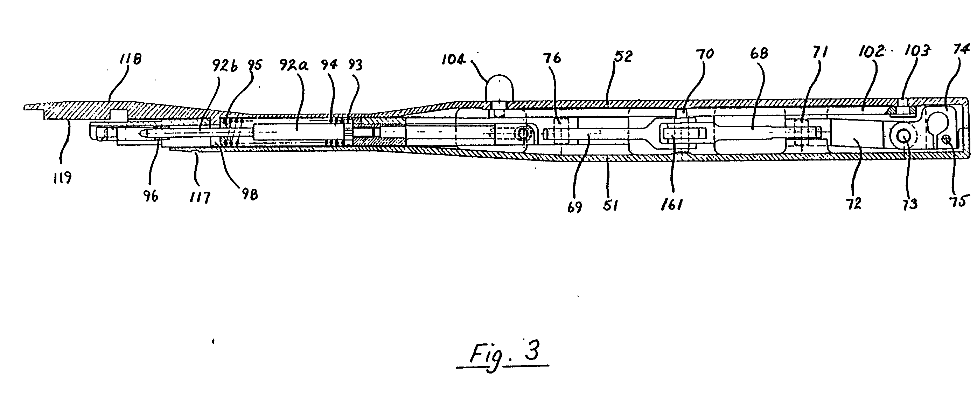

The illustrated instrument shown generally in Fig. 1 comprises a housing 50 made up, as shown in Fig. 3, of a left-hand housing half 51 and a right-hand housing half 52. Detachably mounted at the forward end of the instrument housing 50, with the aid of a locking slide 53, is a jaw member 54 comprising respective forward and rear jaws 55 and 56 and a bight portion 57. Pivotally mounted on the rear portion of the housing 50 is an operating lever 58.

The housing halves 51 and 52 are held together at the rear and at the central portions of the instrument, by means of pivoting latches 59 which will be described in detail below. At the forward end of the instrument housing 50, the halves 51 and 52 are held together by the locking slide 53 as described hereinafter.

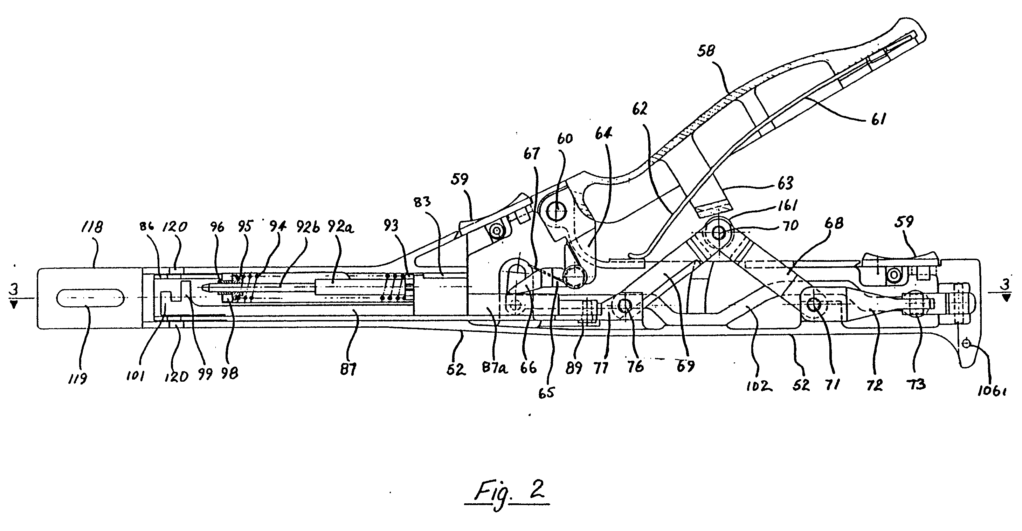

The operating lever 58 is mounted on a pivot pin 60 the ends of which are located in the respective housing halves 51 and 52. A leaf spring 61, the free end 62 of which bears on the upper surface of the housing 50, biases the lever 58 to its open position as illustrated in Fig. 2. Extending downwardly from the central portion of the lever 58 is a toggle driving member 63, while depending from the forward end of the lever 58 is a member 64 on the lower end of which is pivotally mounted a pawl 65, having a downwardly and forwardly extending nose portion 66. The pawl 65 is biased downwardly by means of a spring 67.

Lying within the housing 50 is a toggle comprising a rear toggle link 68 and a forward toggle link 69, these links being interconnected by a toggle pin 70. This toggle pin carries a roller 161 for engagement by the toggle driving member 63 as described below. The rear toggle link 68 is connected by means of a pin 71 with a member 72 the rear end of which is connected, by means of a pin 73, with a pressure plate 74 fixed to the housing half 52 by means of a pin 75. The function of the plate 74 will be described below.

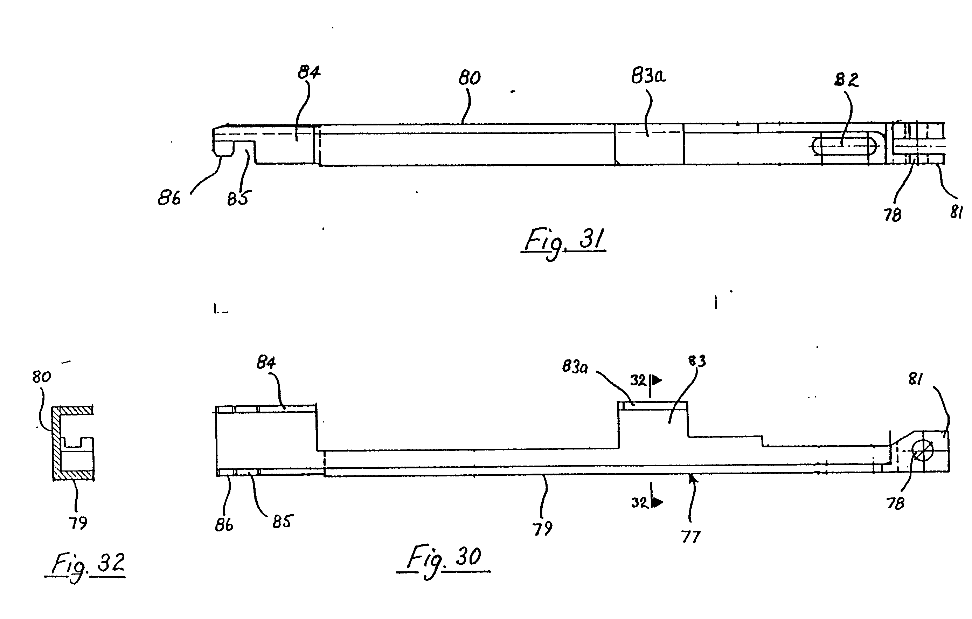

At its forward end, the forward toggle link 69 is connected by means of a pivot pin 76 with the rearward end of a pusher member 77, the pin 76 passing through apertures 78 in the rear end of the pusher 77, which is shown in detail in Figs. 30 to 32.

The pusher 77 is shaped and dimensioned for longitudinal sliding movement within the housing 50, and comprises a base 79 and a side wall 80. The apertures 78 are provided respectively in the side wall 80 and in a web 81, and an elongated slot 82 is provided in the base 79 of the pusher 77 for a purpose described below.

In the middle region of the pusher 77, the side wall 80 is extended upwardly by web 83 and a flange 83a extends inwardly from this extended portion of the side wall, for a purpose to be described below. Similarly, at the forward end of the pusher 77 the side wall 80 is again extended upwardly, and a flange 84 extends laterally from this portion. The flange 84 and the underlying portion of the base 79 are similarly formed with slots 85 and inwardly directed lugs 86.

Mounted within the pusher 77 for longitudinals sliding movement relative thereto, is a T-bar extension 87, shown in detail in Figs. 27 to 29. At the rear end of the T-bar extension 87 a vertical hole 88 is provided, for the reception of a pin 89 (Fig. 2). This pin has an enlarged head and passes through the slot 82 in the base 79 of pusher 77, the head of the pin lying below the base 79, this pin thereby functioning to retain the T-bar extension 87 in assembly with the pusher 77, the slot 82 allowing for the necessary relative longitudinal movement of the T-bar extension 87.

Forward of the pin 89, the T-bar extension 87 is provided with a portion 87a of increased height, to form a rearwardly facing shoulder 87b, the purpose of which will be explained below.

The T-bar extension 87 is also provided near the middle thereof, with a raised portion containing a drilled hole 90. This raised portion is dimensioned for a neat fit beneath the flange 83 of the pusher 77, and is provided at its forward end with a shoulder 91 and 91a which bear against the forward edges of the web 83 and the flange 83a to limit the rearward relative movement of the T-bar extension 87 with respect to the pusher 77.

The hole 90 is tapped to receive the threaded rear end of a stop pin 92 comprising a body 92a and a forwardly extending spring guide portion 92b. By virtue of this screwed connection with the T-bar extension 87, the stop pin 92 is longitudinally adjustable, and is locked in its desired position by means of a lock nut 93 which bears against the surface of the T-bar extension 87 surrounding the threaded hole 90. Surrounding the stop pin 92 is a helical compression return spring 94, the rearward end of which bears against the lock nut 93 and the forward end of which bears against a spring retainer 95 which is slidably mounted on the forward portion of the stop pin 92. To prevent the escape of the return spring 94 and the spring retainer 95 from the stop pin when the instrument is disassembled for cleaning, a retaining nut 96 is screwed on to the threaded forward end of the stop pin 92.

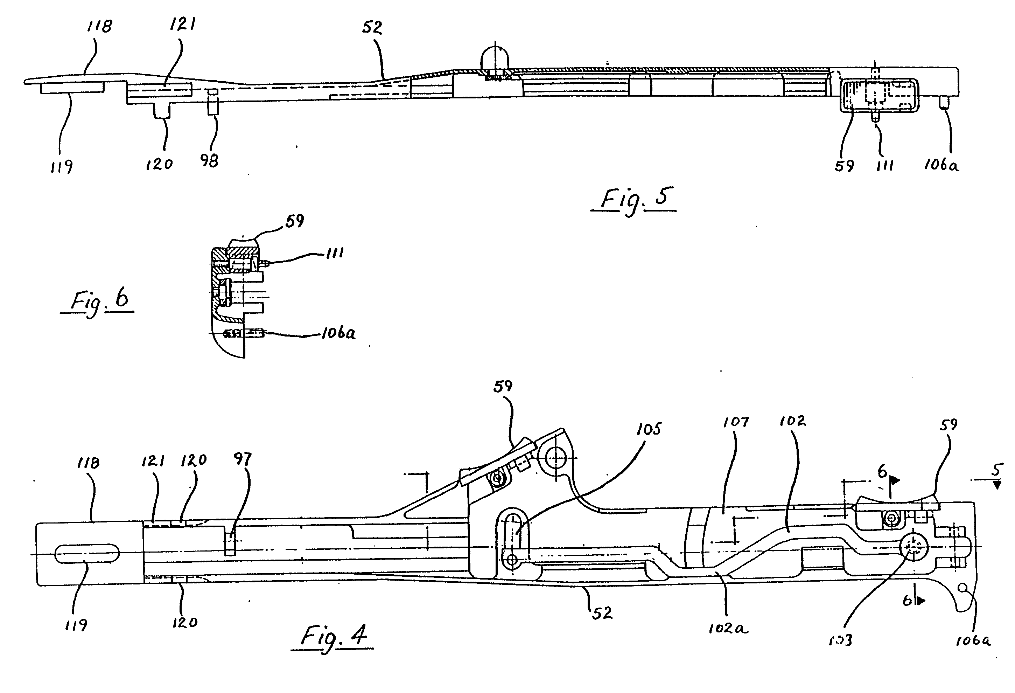

The forward end of the stop pin 92 passes through a slot 97 in a stop lug 98 (Figs. 4 and 5) which extends inwardly from the upper portion of the side wall of the right-hand housing half 52. The slot 97 is open at the left-hand extremity of the lug 98, so that the stop pin and spring assembly may be freed from the housing when the drive assembly is removed for cleaning.

At the forward end of the T-bar extension 87 an upwardly extending lug 99 is separated by a slot 100 from a further upwardly extending lug 101 at the extreme forward end of the T-bar extension.

Mounted within the rear portion of the housing 50 is a release lever 102. This lever, which is best seen in Figs. 3 and 4, is pivotally fixed at its rear end to the right-hand housing half 52 by means of a pivot 103. At the forward end of the lever 102, a release lever button 104 passes through an arcuate slot 105 in the right-hand housing half 52. In the central region of the release lever 102 there is provided a depressed portion 102a which lies below the right-hand end of the toggle pin 70, which is extended beyond the toggle members towards the right-hand housing half 52.

Apart from the latches 59 referred to above, engagement of the housing halves is guided by means of a locating pin 106a anchored in an aperture in the right hand housing half 52 and adapted to enter an aperture 106 in the other housing half. The upper wall of each housing half 51 and 52 is open at 107 to provide clearance for the toggle links 68 and 69 when that toggle is in its contracted condition as illustrated in Fig. 2.

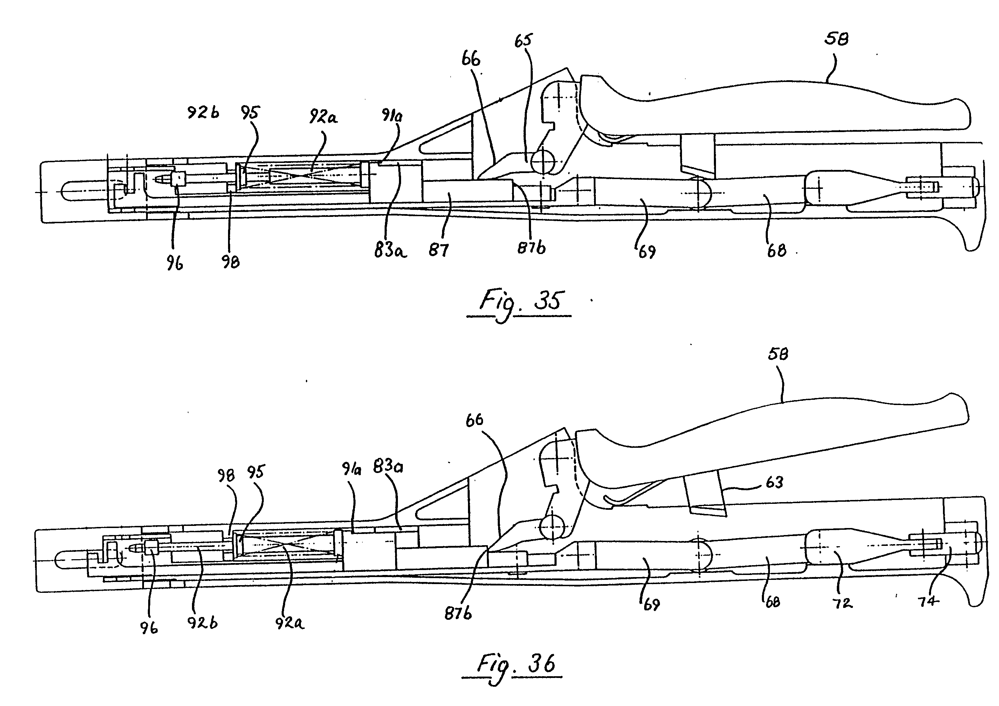

The latches 59 are illustrated in Figs. 33 and 34, and comprise a housing portion 108 the upper surface of which is concavely contoured for ease of operation with the thumb or finger, and depending donwwardly from the housing 108 there is provided a lug 109 which is provided with a drilled hole 110 for the reception of a pivot pin 111 (Fig. 6), one end of which is anchored in the right-hand housing half 52, and the other end of which is located in an aperture 112 in the left-hand housing half. Towards one end of the housing 108, the latch is provided with a pair of opposed downwardly extending lugs 113.

The upper wall of each housing half 52 and 53 is apertured at 114 to allow for rotation of the latch on its pivot pin 111, and rearward of this aperture 114 there is provided a slot 115 for the reception of one of the lugs 113. These apertures 115 are provided in flanges 116 located below the upper surface of the respective housing half, by a distance corresponding to the base thickness of the latch housing 108. As will be appreciated, when the housing halves are correctly position together, each latch may be rotated from its open position to a closed position in which the lugs 113 engage within the respective apertures 115 of the adjacent housing halves, locking the housing halves together. As will be seen in Fig. 34, the inner surfaces of the lugs 113 are tapered to facilitate this locking action.

Adjacent the forward end of the left-hand housing half 51 is a vertical groove 117. The forward end of the right-hand housing half extends beyond that of the left-hand housing half in the region indicated at 118, this region having no bottom or upper wall. On the inner surface of this portion 118, there is provided a longitudinally directed key 119. Rearwardly of the portion 118, from the upper and lower walls of the right-hand housing half 52, there are provided inwardly directed lugs 120, and in the region of these lugs, the outer surfaces of the upper and lower walls of the right-hand housing half 52 are relieved to form grooves 121. The lugs 120 are located so that their rear edge abuts the front edge of the left-hand housing half 51, and extends laterally to an extent equal to the width of the left-hand housing half 51 at its forward end. The purpose of the key 119 and the grooves 121 is to enable locking assembly of the housing of the instrument with the head assembly, which will now be described.

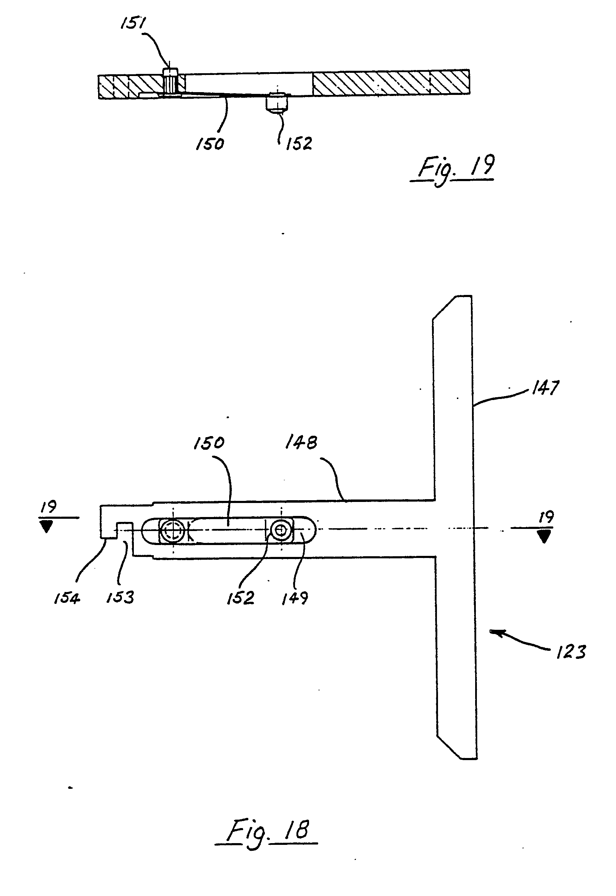

The head assembly of the instrument basically comprises the jaw member 54, a cartridge holder 122 and a T-bar 123. The T-bar is illustrated in Figs. 18 and 19 in a reversed position relative to the remainder of the components illustrated, in order that its locking features to be described below, may best be shown.

The jaw sections of the jaw member 54, comprising the forward jaw 55, rear jaw 56 and bight portion 57 have already been described. As in the prior art, the inner face of the forward jaw 55 is provided with an anvil receiving formation 124. An aperture 125 in the upper end of the forward jaw 55 receives the forward end of a tie pin 126, the rearward portion of which is passed through a cylindrical insert 127 which is held between flanges 128 which stand upwardly at the upper end of the rear jaw 56.

A guide rail formation 129 is provided in the forward region of the upper surface of the bight portion 57, for the purpose of guiding and laterally locating the bottom of the cartridge holder 122.

The upper and lower portions of the rear jaw 56 are joined by a web 130 provided with upper and lower flanges 131. The web 130 and the flanges 131 extend rearwardly from the jaw 56, and the web 130 is provided with a longitudinally orientated slot 132, this slot being dimensioned neatly to receive the key 119 provided on the forward portion 118 of the housing portion 52. The flanges 131 extend, in part, rearwardly for a short distance beyond the web 130, and vertically aligned slots 133 are provided at the left-hand side of the extending portions of these flanges 131. The upper and lower surfaces of the flanges 131 are relieved to provide grooves 134 which open to the rear edge of the flanges 131, in the region 135 where the flanges terminate co-extensively with the web 130. These grooves 134 are located so as to align with the grooves 121 in the forward end of the right-hand housing half 52, and the formations described are dimensioned such that when the jaw member 54 is engaged with the instrument housing, the key 119 enters the slot 132, the portion 135 abuts the forward end of the upper and lower walls of the right-hand housing half 52, and the rearmost edge of each flange 131 abuts the forward edge of the respective lug 120.

The cartridge holder 122 is of generally T-shape in side elevation, comprising a pair of head flanges 136 provided with opposed grooves 137 for the reception of a staple cartridge in the manner known in the prior art. Extending rearwardly from the head flanges 136 is a stem 138 comprising upper and lower walls 139 and 140 respectively, left-hand side wall 141 and a longitudinally slotted right-hand side wall 142. The slot 143 in the right-hand side wall 142 extends throughout the length of that side wall and continues forwardly through portion of the right-hand head flange 136. On the inner surface of the left-hand side wall 141 there is provided a groove 144 which runs for the entire length of this side wall and continues for the entire longitudinal extent of the left-hand head flange 136. On the outer surface of the left-hand side wall 141 forwardly of the rear thereof, there is provided a vertical groove 145, and the rear portion of the right-hand side wall is removed to allow the formation in the upper and lower walls 139 and 140, of a slot 146 and lug 146a (Fig. 16). These formations are dimensioned and located such that upon engagement of the head assembly with the instrument housing, the lugs 146 enter the slots 85 at the forward end of the pusher 77, and the lugs 86 at the forward end of the pusher 77 enter the slots 145.

As illustrated in Figs. 18 and 19, the T-bar 123 comprises a head 147 and a stem 148. The stem 148 is slotted at 149 and in this region a leaf spring 150 is provided, mounted at its rear end by means of a pin 151. The leaf spring 150 is biased outwardly of the slot 149 towards the right-hand side of the T-bar, and at its forward end there is mounted a locking pin 152. The locking pin 152, in the relaxed condition of the spring 150, stands proud of the right-hand side of the T-bar 123. The T-bar is, of course, mounted within the cartridge holder 122, with its head 147 between the head flanges 136 of the cartridge holder, and its stem 148 within the stem 138 of the cartridge holder. The pin 151 extends beyond the left-hand side of the T-bar and runs in the groove 144 in the left-hand side wall of the cartridge holder, while the locking pin 152 is free to lie within the slot 145 in the right-hand side wall 142 of the cartridge holder stem 138, and projecting outwardly beyond that side wall.

At the rearward end of the stem 148 there is provided a slot 153 and a downwardly directed lug 154, the lug 154 being located and dimensioned to engage with the slot 100 in the forward end of the T-bar extension 87 upon engagement of the head assembly with the instrument housing.

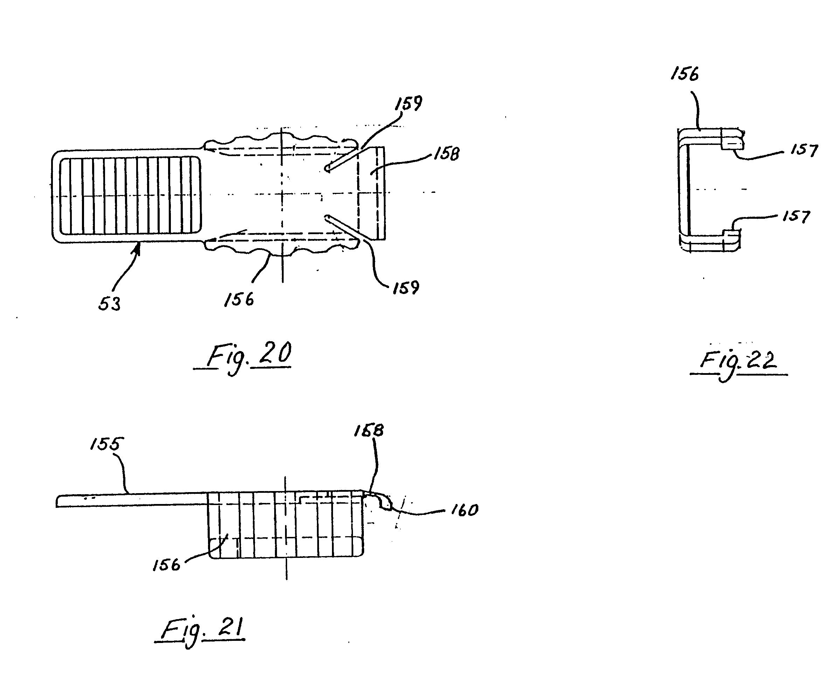

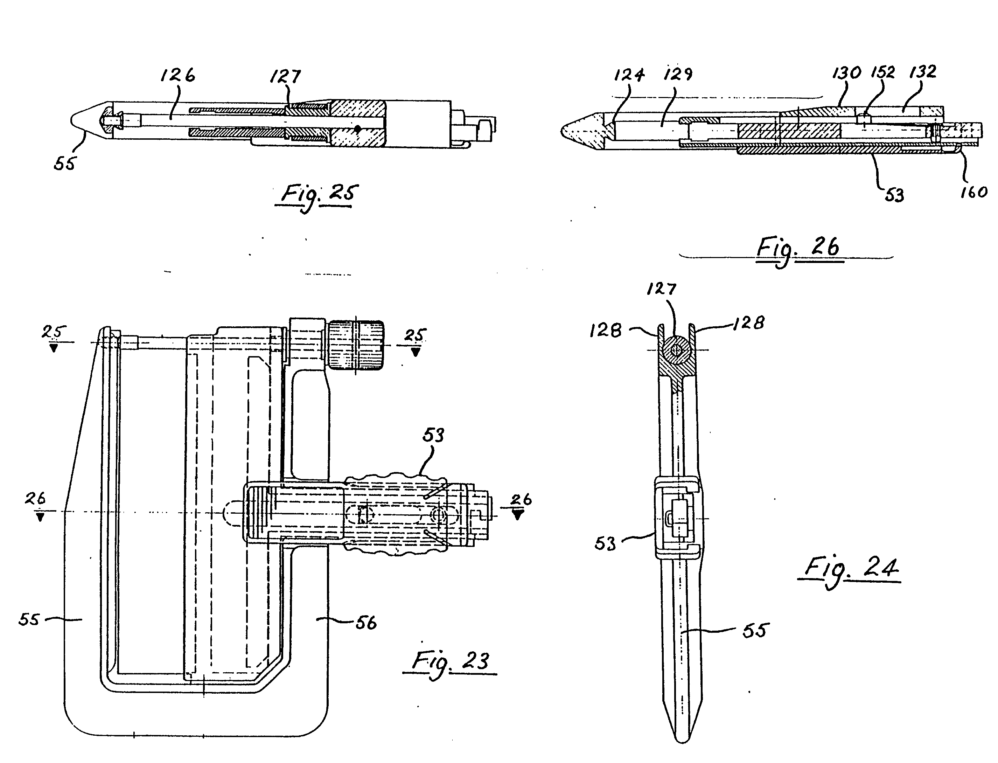

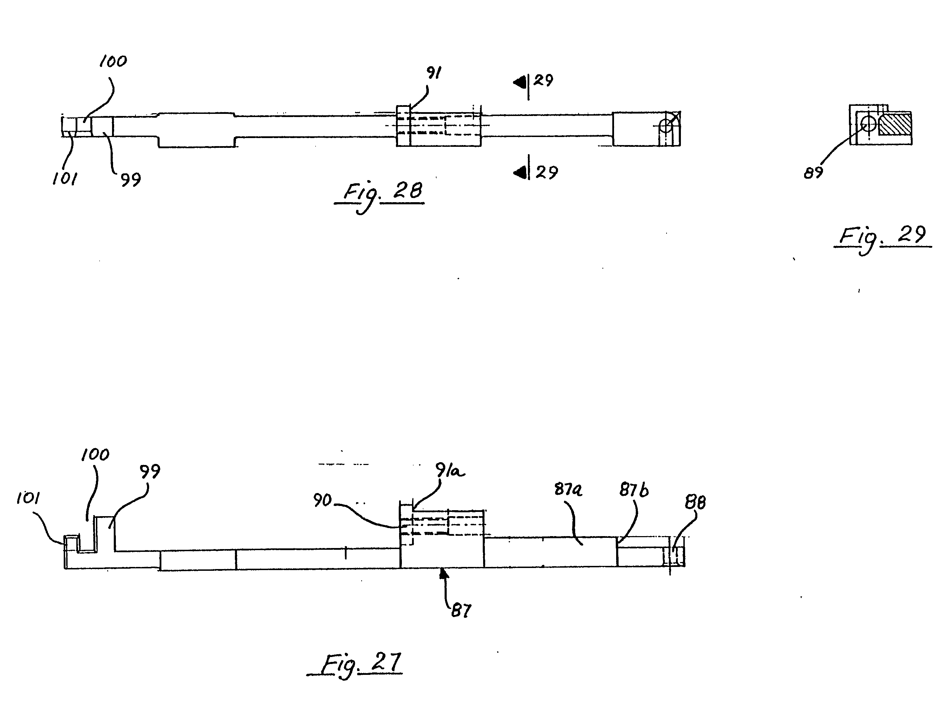

The head assembly is completed by the locking slide 53, which is illustrated in Figs. 20 to 22. The locking slide 53 comprises a web 155 and a pair of flanges 156. The outer surface of the flanges 156 is provided with vertical grooves to assist the operator's grip, and each flange 156 is provided with opposed inwardly directed flanges 157.

A resilient tongue 158 is formed at the rear end of the web 155, by means of diagonal slots 159, and the rear edge of the tongue 158 is provided with an inwardly directed detent 160.

Figs. 23 to 26 show the relationship between the components of the head assembly, in their assembled condition prior to engagement of the head assembly with the remainder of the instrument. The locking slide 53 embraces the stem 138 of the cartridge holder 122, with the flanges 157 engaged within the grooves 134, while the detent 160 is snapped into the groove 133. The locking pin 152 of the T-bar 123 extends through the slot 145 in the cartridge holder stem 138 to enter the slot 132 in the web 130, thereby restricting longitudinal movement of the T-bar in this condition.

The immediately apparent advantage of this arrangement by which the components of the head assembly are held in their correct relative positions is the facilitation of correct engagement of the head assembly with the remainder of the instrument. Other advantages flow however, in particular the instrument described enables the surgeon to position the head assembly in relation to the patient before attaching the housing and drive mechanism, and it will be appreciated that this offers advances in the surgical techniques and increases the utility of the instrument.

The head assembly is engaged with the instrument housing and actuating mechanism, with the T-bar 123 in its forwardmost position as defined by the engagement of the locking pin 152 with the forward end of the slot 132, by longitudinally aligning the housing and head assembly and moving the latter from the left such that the key 119 enters the slot 132. As this occurs the T-bar extension 87 will engage with the T-bar 123 and the pusher 77 will engage with the cartridge holder 122 as previously described, and the key 119 will press the T-bar locking pin 152 inwardly and out of the slot 132, freeing the T-bar for operative longitudinal movement. The operator now grips the locking slide 53 and moves it rearwardly, so that the flanges 157 move into the grooves 121 and the flanges 156 embrace the upper and lower walls of both housing parts 51 and 52, and the flanges 131 of the jaw member 54. The housing parts 51 and 52 are pressed together by the web 155.

The locking slide 53 is drawn on to the housing until the detent 160 engages the groove 117 in the left hand housing part 51. With the mounting of a cartridge and anvil in known manner, the instrument is then ready for use.

The operation of the instrument thus described is as follows, and reference should be made to Figs. 2, 23 and 24. In Fig. 2 the mechanism is shown in its initial condition. Tissue proximation is first achieved by depressing the operating lever 58 so that the toggle drive member 63 contacts the toggle centre roller 161, driving this downwardly and thereby driving the pusher 77 forward by virtue of its engagement with the forward toggle pin 76. The cartridge holder 122 is thus moved to the staple firing position.

The return spring 94 urges the T-bar extension rearwardly so that its shoulder 91 is urged against the pusher flange 83. The T-bar extension, and thus the T-bar, will therefore move forward with the pusher.

The mechanism will now be in the position shown in Fig. 35. During this movement, the pawl 66 also moves forwardly, but with no function.

When the operating lever 58 is released and allowed to return to its open position with the aid of the leaf spring 61, the pawl 66 will return to its rearmost position, and the pusher and T-bar extension will remain in their forward position due to the toggle 68 and 69 having been driven over-centre.

With the T-bar extension 87 thus moved forward, the returning pawl 66 will drop behind the T-bar extension shoulder 87b. On the second depression of the operating lever therefore, the pawl 66 will engage this shoulder and drive the T-bar extension 87, and thus the T-bar 123, forward, producing staple firing. This position of the mechanism is illustrated in Fig. 36.

Forward motion of the T-bar extension will be arrested when the shoulder formed by the forward end of the stop pin housing 92a is driven up against the spring retainer 95, which bears against the stop lug 98. As mentioned above, this limit of travel is adjustable by virtue of the screwed mounting of the stop pin 92.

As will no doubt be appreciated, the function of the pressure plate 74 is to absorb the effects of excessive tissue thickness or other variations which may effect the stapling force and staple formation. This member acts as a compression spring, allowing the entire drive mechanism to move backwards under excessive stapling force, and thereby increasing the effective tissue gap.

Other types of residual devices may be substituted for the pressure plate 74, such as constant-rate springs.

Thus the steps of tissue proximation and staple firing are carried out by successive movements of the one operating member, considerably simplifying the operation of the instrument. This action, combined with the disposition of the operating lever 58, enables true one-handed operation to be achieved.

The sequence of operations is completed by the operator moving upwardly (as viewed in the drawings) the release button 104. This raises the release lever 102 as it pivots about the pin 103, and the portion 102a contacts the extended portion of the toggle pin 70, forcing the toggle through its centre of motion, whereupon the pressure of the return spring on the T-bar extension 87 and the pusher 77 will return the toggle to its initial position.

The drive mechanism thus described has a further advantage which arises from the fact that the pusher, T-bar extension, toggle links, and pivot link 72 will remain interconnected when lifted away from the right-hand housing half 52 for cleaning, the pivot link 72 enabling this movement while retaining the connection of these components to the housing half. Thus the mechanism may be cleaned between actuations with the minimum delay in re-assembly, such re-assembly requiring no special techniques and no tools. It will be appreciated that the use of latches 59 to retain the housing halves together, in cooperation with the locking slide 53, also greatly simplifies the partial disassembly of the instrument for cleaning.

The ease with which head assemblies may be interchanged in the instrument of the present invention enables such head assemblies, comprising jaws, cartridge holder, T-bar and cartridge to be disposable, should this be desired.

It will be appreciated that while one embodiment of the invention has been described in detail, the purpose of this description is solely to show the best manner of performance of the principles of the invention presently known, and it will be understood that many variations and alternative mechanical arrangements by which these principles may be realised are possible.

## Claims

1. A surgical stapling instrument of the kind in which staples are ejected from a staple cartridge to be formed against an anvil surface mounted in justaposition with said cartridge, said cartridge being movable between a staple firing position at a predetermined distance from said anvil surface and a retracted position at a greater distance from said anvil surface, said instrument comprising a cartridge holder (122) movable to control the position of said cartridge and a staple driving member movable towards said cartridge to cause ejection of staples therefrom and an operating lever (58) movable to cause staple ejecting motion of said staple driving member, wherein said operating lever is operable in a first movement thereof to cause movement of said cartridge holder towards said anvil to place said cartridge in said staple firing position and is operable in a second movement thereof to cause said staple ejecting motion of said driving member and pusher means (77) extending rearwardly from said cartridge holder and longitudinally moveable to control the position of the cartridge holder, pusher drive means (68-70) actuated by said operating lever to move said pusher means in a forward direction to place said cartridge holder in said staple firing position, staple driving member extension means (87) extending rearwardly from said staple driver, and a staple firing member associated with said operating lever, said staple firing member being engagable with said extension means after actuation of said pusher drive means; characterised in that said pusher drive means comprises a toggle (68, 69, 70) having a rear link (68) and a forward link (69), the forward pivot (76) of said forward link being connected to said pusher means, the forward and rear links being interconnected by a toggle pivot (70), said operating lever being provided with means (63) engaging said toggle in the region of said toggle pivot upon movement of said lever in an operating direction to expand said toggle thereby moving said pusher means (77) forwardly.

2. A surgical stapling instrument according to Claim 1 further characterised in that said toggle pivot (70) moves over-centre when fully actuated by said operating lever.

3. A surgical stapling instrument according to Claim 2 further characterised in that return of said operating lever to its unactuated position does not affect said toggle.

4. A stapling instrument according to Claim 3 further characterised in that said instrument comprises toggle release means (102, 104) operable to return said toggle pivot from said over-centre position.

5. A stapling instrument according to Claim 1 further characterised in that said staple firing member comprises pawl means (65) mounted for movement with said operating lever such as to move forwardly upon actuating movement of said lever and rearwardly upon movement of said lever to its unactuated position, said extension means moving forwardly with said pusher means, pawl engagement means (87b) being provided on said extension means (87) engagable by said pawl means when said cartridge holder is in said firing position and said operating lever is substantially in its unactuated position.

6. A stapling instrument according to any preceding Claim further characterised in that said instrument comprises a return spring (94) biasing said extension means and said pusher means rearwardly toward their unactuated positions.

7. A stapling instrument according to Claim 1 further characterised in that said rear pivot (71) is connected to said housing by resilient means (74) such that said pivot is capable of limited rearward movement in conditions of excessive stapling force.

8. A surgical stapling instrument according to Claim 1 further characterised in that the pusher means, driving member extension means and pusher drive means are interconnected as a subassembly and fixed within said housing only at the rear of said sub-assbembly by means enabling the pivotal displacement of said subassembly outwardly of said housing.

9. A surgical stapling instrument according to Claim 7 or Claim 8 wherein said means enabling pivotal displacement comprises a link (72) pivotally connected between said resilient means and said rear pivot.

**Patentansprüche**

1. Chirurgisches Heftinstrument des Typs, bei welchem Heftklammern aus einer Klammerpatrone zur Formung an einer in Juxtaposition zur Patrone gelagerten Amboßfläche ausgestoßen werden, wobei die Patrone zwischen einer Klammer-Feuerstellung in einem bestimmten Abstand von der Amboßfläche und einer Rückzugstellung in einem größeren Abstand von der Amboßfläche beweglich ist, welches Instrument einen zur Steuerung der Position der Patrone beweglichen Patronenhalter (122) und ein zur Patrone bewegliches Klammerführungselement zum Bewirken des Ausstoßes der Klammern aus diesem und einen zum Bewirken der Klammerausstoßbewegung beweglichen Betätigungsarm (58) des Klammerführungselements umfaßt, wobei der Betätigungshebel in eine erste Bewegung betätigbar ist, um die Bewegung des Patronenhalters zum Amboß zwecks Versetzens der Patrone in die Klammer-Feuerstellung und in eine zweite Bewegung zwecks Bewirkens der Klammerausstoßbewegung des Führungselements betätigbar ist, und ein sich vom Patronenhalter nach hinten erstreckendes und zwecks Steuerung der Position des Patronenhalters in Längsrichtung bewegliches Schubmittel (77), ein vom Betätigungshebel betätigtes Schubantriebsmittel (68-70) zum Bewegen des Schubmittels in Vorwärtsrichtung zwecks Versetzens des Patronenhalters in die Klammer-Feuerstellung, ein sich von der Klammerführung nach hinten erstreckendes Verlängerungsmittel (87) des Klammerantriebselements und ein mit dem Betätigungshebel in Verbindung stehendes Klammerfeuerungselement umfaßt, wobei das Klammerfeuerungselement nach Betätigung des Schubantriebsmittels mit dem Verlängerungsmittel in Eingriff bringbar ist; dadurch gekennzeichnet, daß das Schubantriebsmittel ein Kniegelenk (68, 69, 70) mit einer hinteren Schwinge (68) und einer vorderen Schwinge (69) umfaßt, wobei der vordere Schwenkzapfen (76) der vorderen Schwinge mit dem Schubmittel verbunden ist und die vordere und die hintere Schwinge durch einen Kniegelenkzapfen (70) miteinander verbunden sind, wobei der Betätigungshebel mit einem Mittel (63) versehen ist, welches im Bereich des Kniegelenkzapfens bei Bewegung des Hebels in Betätigungsrichtung zum Ausstrecken des Kniegelenks und dadurch Bewegung des Schubmittels (77) nach vorne am Kippglied angreift.

2. Chirurgisches Heftinstrument nach Anspruch 1, weiters dadurch gekennzeichnet, daß sich der Kniegelenkzapfen (70) bei voller Betätigung des Betätigungshebels über die gestreckte Lage hinaus bewegt.

3. Chirurgisches Heftinstrument nach Anspruch 2, weiters dadurch gekennzeichnet, daß die Retourbewegung des Betätigungshebels in seine unbetätigte Position das Kniegelenk nicht beeinflußt.

4. Heftinstrument nach Anspruch 3, weiters dadurch gekennzeichnet, daß das Instrument ein Kniegelenk-Freigabemittel (102, 104) umfaßt, das zum Zurückbringen des Kniegelenkzapfens aus der Position über die gestreckte Lage betätigbar ist.

5. Heftinstrument nach Anspruch 1, weiters dadurch gekennzeichnet, daß das Klammerfeuerungselement ein Klinkenmittel (65) umfaßt, welches zur Bewegung mit dem Betätigungshebel angeordnet ist, sodaß es sich bei Betätigung der Bewegung des Hebels nach vorne bewegt und bei Bewegung des Hebels in seine unbetätigte Position nach hinten bewegt, wobei das Verlängerungsmittel sich mit dem Schubmittel nach vorne bewegt und ein Klinkeneinrastmittel (87b) auf dem Verlängerungsmittel (87) vorgesehen ist, das mit dem Klinkenmittel in Eingriff bringbar ist, wenn sich der Patronenhalter in der Feuerstellung und der Betätigungshebel in im wesentlichen seiner unbetätigten Position befinden.

6. Heftinstrument nach einem vorhergehenden Anspruch, weiters dadurch gekennzeichnet, daß das Instrument eine Rückholfeder (94) umfaßt, die das Verlängerungsmittel und das Schubmittel nach hinten in Richtung zu ihren unbetätigten Positionen drückt.

7. Heftinstrument nach Anspruch 1, weiters dadurch gekennzeichnet, daß der hintere Schwenkzapfen (71) durch ein nachgiebiges Mittel (74) derart mit dem Gehäuse verbunden ist, daß der Schwenkzapfen unter Bedingungen übermäßiger Heftkraft zu einer begrenzten Rückwärtsbewegung fähig ist.

8. Chirurgisches Heftinstrument nach Anspruch 1, weiters dadurch gekennzeichnet, daß das Schubmittel, das Verlängerungsmittel des Antriebselements und das Schubantriebsmittel als Untergruppe miteinander verbunden sind und innerhalb des Gehäuses nur am hinteren Teil der Untergruppe durch ein Mittel befestigt sind, das die Schwenkbewegung der Untergruppe nach außerhalb des Gehäuses ermöglicht.

9. Chirurgisches Heftinstrument nach Anspruch 7 oder Anspruch 8, worin das Mittel zum Ermöglichen der Schwenkbewegung eine schwenkbar zwischen dem nachgiebigen Mittel und dem hinteren Schwenkzapfen angeschlossene Schwinge (72) umfaßt.

**Revendications**

1. Instrument d'agrafage chirurgical du genre dans lequel des agrafes sont éjectées d'une cartouche d'agrafes pour être façonnées contre une surface d'enclume montée en juxtaposition à ladite cartouche, ladite cartouche étant mobile entre une position d'expulsion d'agrafe située à une distance déterminée de ladite surface d'enclume et une position rétractée plus distante de ladite surface d'enclume, ledit instrument comprenant un porte-cartouche (122) mobile pour régler l'emplacement de ladite cartouche, un organe d'enfoncement d'agrafe mobile vers ladite cartouche pour provoquer l'éjection d'agrafes hors de celle-ci et une manette (58) mobile pour provoquer le déplacement d'éjection d'agrafe dudit organe d'enfoncement d'agrafe, dans lequel ladite manette agit lors d'un premier mouvement qu'elle décrit pour provoquer le déplacement dudit porte-cartouche vers ladite enclume afin de placer ladite cartouche dans ladite position d'expulsion d'agrafe et agit lors d'un second mouvement qu'elle décrit pour provoquer ledit déplacement d'éjection d'agrafe dudit organe d'enfoncement et un poussoir (77) s'étendant vers l'arrière à partir dudit porte-cartouche et mobile longitudinalement pour régler l'emplacement du porte-cartouche, un moyen d'entraînement de poussoir (68, 70) actionné par ladite manette pour déplacer ledit poussoir vers l'avant afin de placer ledit porte-cartouche dans ladite position d'expulsion d'agrafe, un moyen de prolongement d'organe d'enfoncement d'agrafe (87) s'étendant vers l'arrière à partir dudit organe d'enfoncement d'agrafe et un organe d'expulsion d'agrafe associé à ladite manette, ledit organe d'expulsion d'agrafe pouvant rencontrer ledit moyen de prolongement après actionnement dudit moyen d'entraînement de poussoir; caractérisé en ce que ledit moyen d'entraînement de poussoir est un tringlage articulé (68, 69, 70) comportant une tringle arrière (68) et une tringle avant (69), le tourillon avant (76) de ladite tringle avant étant relié audit poussoir, les tringles avant et arrière étant reliées l'une à l'autre par un tourillon de tringlage articulé (70), ladite manette étant munie d'un moyen (63) portant contre ledit tringlage articulé dans la région dudit tourillon de tringlage articulé lors du déplacement de ladite manette dans le sens de manoeuvre pour déployer ledit tringlage articulé déplaçant par là ledit poussoir (77) vers l'avant.

2. Instrument d'agrafage chirurgical selon la revendication 1 caractérisé en outre en ce que ledit tourillon de tringlage articulé (70) franchit une position d'équilibre lorsqu'il est actionné à fond par ladite manette.

3. Instrument d'agrafage chirurgical selon la revendication 2 caractérisé en outre en ce que retour de ladite manette en position de non actionnement n'affecte pas ledit tringlage articulé.

4. Instrument d'agrafage chirurgical selon la revendication 3 caractérisé en outre en ce que ledit instrument comprend un moyen de libération de tringlage articulé (102, 104) assurant le rappel du tourillon de tringlage articulé à partir de ladite position de franchissement de point d'équilibre.

5. Instrument d'agrafage chirurgical selon la revendication 1 caractérisé en outre en ce que ledit organe d'expulsion d'agrafe est un cliquet (65) monté de façon à se déplacer avec ladite manette en sorte de se déplacer vers l'avant lors du déplacement actif de ladite manette et vers l'arrière lors du déplacement de ladite manette jusqu'à sa position inactive, ledit moyen de prolongement se deplaçant vers l'avant avec ledit poussoir, un moyen d'appui de cliquet (87b) étant prévu sur ledit moyen de prolongement (87) pour être rencontré par ledit cliquet quand ledit porte-cartouche est dans ladite position d'expulsion et que ladite manette est sensiblement dans sa position de non actionnement.

6. Instrument d'agrafage selon toute revendication précédente caractérisé en outre en ce que ledit instrument comprend un ressort de rappel (94) sollicitant ledit moyen de prolongement et ledit poussoir vers l'arrière en direction de leurs positions de non actionnement.

7. Instrument d'agrafage chirurgical selon la revendication 1 caractérisé en outre en ce que ledit tourillon arrière (71) est relié audit logement par un moyen élastique (74) de sorte qu'il peut décrire un mouvement vers l'arrière limité en cas de force d'agrafage excessive.

8. Instrument d'agrafage chirurgical selon la revendication 1 caractérisé en outre en ce que le poussoir, le moyen d'entraînement de l'organe de prolongement et le moyen d'entraînement de poussoir sont reliés entre eux en tant que sous-ensemble et fixés à l'intérieur dudit logement seulement à l'arrière dudit sous-ensemble par un moyen permettant le déplacement pivotant dudit sous-ensemble vers l'extérieur dudit logement.

9. Instrument d'agrafage chirurgical selon la revendication 7 ou la revendication 8 dans lequel ledit moyen permettant un déplacement pivotant est une tringle (72) articulée entre ledit moyen élastique et ledit tourillon arrière.

*Fig. 1*

*Fig. 33*

*Fig. 34*

_Fig. 2_

EP 0 096 694 B1

Fig. 3

3

Fig. 5

Fig. 6

Fig. 4

_Fig. 8_

_Fig. 7_

_Fig. 9_

_Fig. 12_

_Fig. 10_

_Fig. 11_

_Fig. 13_

Fig. 17

Fig. 14

Fig. 16

Fig. 15

141  144  145

145  141  138  122  136

146  146a  145  139  136

136  144  136

_Fig. 19_

_Fig. 18_

Fig. 22

Fig. 20

Fig. 21

126    127

55

*Fig. 25*

124  129                    130  152  132

53        160

*Fig. 26*

127

128                128

53

55

*Fig. 24*

25                    25

26                    26

53

55                    56

*Fig. 23*

EP 0 096 694 B1

Fig. 29

Fig. 28

Fig. 27

EP 0 096 694 B1

84    80    83a    82

86    85    78    81

*Fig. 31*

84    83a    32    83    81

86    85    79    77    78

80

79

*Fig. 32*    *Fig. 30*

EP 0 096 694 B1

92b  95      92a     91a

96   98      83a  87      87b      69      68

66   65                          58

Fig. 35

98 95      91a 83a

66                          58

63

96   92b      92a      87b      69      68      72      74

Fig. 36